# EUROPEAN PATENT APPLICATION

(11) **EP 4 166 147 A1**
(43) Date of publication of application: **19.04.2023**
(21) Application number: 21825603.0
(22) Date of filing: 14.06.2021
(51) Int. Cl.: A61K 33/40, A61K 31/728

(54) **SENSITISER FOR CANCER TREATMENT**

(30) Priority: 15.06.2020 JP 2020103121
(71) Applicant: KORTUC Inc., Tokyo 1056004 (JP)
(72) Inventor: NAVITA Somaiah, London, SW36JJ (GB); OGAWA Yasuhiro, Kakogawa-shi, Hyogo 675-0031 (JP)
(74) Representative: Gulde & Partner
(86) International application number: PCT/JP2021/022520
(87) International publication number: WO 2021/256427

(57) **Abstract**

The dose and regimen of the sensitizer of the present invention which is effective for anticancer therapy for tumors and the schedule of the anticancer therapy, such as radiotherapy and anticancer chemotherapy, which is effective after administration of the sensitizer, are still unclear. The present inventors have demonstrated that the sensitizer for anticancer therapy, which is prepared by combining a specific range of concentration of H₂O₂ with a specific range of concentration of hyaluronic acid or a salt thereof in a specific amount, in a specific procedure, can be injected into the affected tumor site to improve the effect of anticancer therapy such as radiation therapy and anticancer chemotherapy, thereby solving the above problems.

## Description

### Technical Field

The present invention relates to a sensitizer for anti-cancer therapy such as radiation therapy or anti-cancer chemotherapy, and especially relates to a sensitizer for anti-cancer therapy comprising hydrogen peroxide and hyaluronic acid or a salt thereof.

### Background Art

Overcoming Breast cancer is a global challenge, with an estimated incidence of breast cancer of 2 million worldwide, 80% of whom present with locally advanced disease (1,2). In the UK, where women with locally advanced disease represent a minority (7-13%) of the 55,000 new patient presentations, the lifetime morbidity of progressive local disease is significant (3-6). Treatment is challenging in frail or elderly individuals who are unfit for or refuse surgery, and for whom RT +/- endocrine therapy is often the most appropriate option for relief of breast ulceration, bleeding, and pain. Locally advanced inoperable primary or recurrent cancers infiltrating the breast/chest wall and/or axilla, with or without metastases, are typically associated with life expectancies measured in years rather than months and present significant challenges to patients and medical professionals. This represents an area of unmet clinical need, where innovative approaches to enhance response to radiation would be highly beneficial.

An interaction at a cellular level between H₂O₂ and ionising radiation (IR) was first reported in osteosarcoma (HS-Os-1) and prostate cancer (PC-3) cell lines, which demonstrated extreme resistance to either H₂O₂ or 30 Gy alone (7,8). The addition of 0.1 mM H₂O₂ prior to IR resulted in enhanced cytotoxicity without causing DNA double strand breaks that classically mediate cell killing (9,10). A novel mechanism was postulated to involve lysosomal membrane rupture with release of powerful oxidants, including heavy metal ions that permeabilise mitochondria and activate apoptosis (11). In vivo use involved a mixture of 0.5% H₂O₂ in 0.83% sodium hyaluronate gel, the Kochi Oxydol-Radiation Therapy for Unresectable Carcinomas (KORTUC) strategy designed to minimise local pain at the injection site. Intratumoral injection of this H₂O₂ gel mixture into murine tumors prior to 30 Gy IR demonstrated clear evidence of growth delay above that achieved by either modality alone. No toxicity was noted (12).

### Citation List

### Non Patent References

[Non-pat Ref. 1] Ginsburg O, Bray F, Coleman MP, et al. The global burden of women's cancers: A grand challenge in global health. Lancet 2017;389:847-860.
[Non-pat Ref. 2] Https://www.Cancer.Org/content/dam/cancer-org/research/cancer-facts-and-statistics/global-cancer-facts-and-figures/global-cancer-facts-and-figures-4th-edition.Pdf.
[Non-pat Ref. 3] Https://www.Cancerresearchuk.Orq/health-professional/cancer-statistics/statistics-by-cancer-type/breast-cancer/incidence-invasive#heading-three.
[Non-pat Ref. 4] Http://www.Ncin.Orq.Uk/publications/survival by stage.
[Non-pat Ref. 5] Http://www.Isdscotland.Org/health-topics/cancer/detect-cancer-early/.
[Non-pat Ref. 6] Http://www.Qub.Ac.Uk/research-centres/nicr/cancerinformation/official-statistics/.
[Non-pat Ref. 7] Ogawa Y, Takahashi T, Kobayashi T, et al. Mechanism of hydrogen peroxide-induced apoptosis of the human osteosarcoma cell line hs-os-1. International journal of molecular medicine 2003;12:459-63.
[Non-pat Ref. 8] Kariya S, Sawada K, Kobayashi T, et al. Combination treatment of hydrogen peroxide and x-rays induces apoptosis in human prostate cancer pc-3 cells. Int J Radiat Oncol Biol Phys 2009;75:449-54.
[Non-pat Ref. 9] Eriksson D, Stigbrand T. Radiation-induced cell death mechanisms. Tumour Biol 2010;31:363-72.
[Non-pat Ref. 10] Katsube T, Mori M, Tsuji H, et al. Most hydrogen peroxide-induced histone h2ax phosphorylation is mediated by atr and is not dependent on DNA double-strand breaks. J Biochem 2014;156:85-95.
[Non-pat Ref. 11] Ogawa Y, Takahashi T, Kobayashi T, et al. Apoptotic-resistance of the human osteosarcoma cell line hs-os-1 to irradiation is converted to apoptotic-susceptibility by hydrogen peroxide: A potent role of hydrogen peroxide as a new radiosensitizer. Int J Mol Med 2003;12:845-50.
[Non-pat Ref. 12] Akima R, Ogawa Y, Morita-Tokuhiro S, et al. New enzyme-targeting radiosensitizer (kortuc) containing hydrogen peroxide & sodium hyaluronate for intratumoral injection using mice transplanted with sccvii tumor. Int J Cancer Clin Res 2016;3:1-6.

### Summary of Invention

### Technical Problem

An effective dosage and usage of a sensitizer for anticancer therapy against tumors and an effective schedule of anticancer therapy such as radiotherapy and anticancer chemotherapy after administration of the sensitizer are still unclear.

### Solution to Problem

The present inventors completed the present invention after intensive research to solve the above problems. That is to say, the present invention provides a sensitizer for anticancer therapy prepared by combining a specific range of concentration of H₂O₂ with a specific range of concentration of hyaluronic acid or a salt thereof, in a specific amount, in a specific procedure, and demonstrates that the sensitizer can improve the effect of anticancer therapy such as radiation therapy and anticancer chemotherapy by injecting into the affected tumor site, thereby solving the above problems.

Specifically, the present invention provides the following embodiments to solve the above-described problems:
[1] A sensitizer for anti-cancer therapy comprising (a) 0.01-3.5 (w/v) % of hydrogen peroxide and (b) 0.1-10 (w/v) % of hyaluronic acid or a salt thereof, wherein the dose of sensitizer administered is selected from the range of 1.2 mL to 12.0 mL in accordance with size of tumor.
[2] The sensitizer according to [1], wherein the sensitizer comprises 0.5 (w/v) % of hydrogen peroxide.
[3] The sensitizer according to [1] or [2], wherein the dose of the sensitizer is determined to:
   (1) the range of 1.2 mL to less than 3.0 mL for a tumor with a maximum diameter of less than 3.0 cm,
   (2) the range of 2.4 mL to less than 6.0 mL for a tumor with a maximum diameter ranging 3.0 cm to less than 6.0 cm,
   (3) the range of 3.6 mL to less than 9.0 mL for a tumor with a maximum diameter ranging 6.0 cm to less than 10.0 cm, and
   (4) the range of 3.6 mL to 15.0 mL for a tumor with a maximum diameter of 10.0 cm or more.
[4] The sensitizer according to any one of [1] to [3], wherein the sensitizer exhibits a pain-reducing effect, a dermatological toxicity-reducing effect, or both the pain-reducing effect and the dermatological toxicity-reducing effect, in addition to the anti-cancer effect.
[5] The sensitizer according to any one of [1] to [4], wherein the anti-cancer therapy is radiation therapy or anti-cancer chemotherapy.
[6] The sensitizer according to any one of [1] to [5], wherein the sensitizer is directly injected into the tumor tissue once to 3 times a week during the tumor treatment period.
[7] The sensitizer according to any one of [1] to [6], wherein the sensitizer is directly injected into the tumor 2 to 8 times in total during the tumor treatment period.
[8] The sensitizer according to any one of [1] to [7], wherein the anti-cancer therapy is performed within 24 hours after the direct injection of the sensitizer into the tumor.
[9] The sensitizer according to [8], wherein the anti-cancer therapy is applied daily.
[10] The sensitizer according to [8] or [9], wherein the dose per fraction in radiation therapy is within the range of 1.75 to 3.75 Gy.
[11] The sensitizer according to [8], wherein the radiation therapy is applied once, twice or three times a week.
[12] The sensitizer according to [11], wherein the dose per fraction in radiation therapy is within the range of 5.0 to 7.0 Gy.

### Advantageous Effects of Invention

The dose and usage of the sensitizer for the anticancer therapy of the present invention can improve the effect of the anticancer therapy (radiotherapy or anticancer chemotherapy) and effectively treat the tumor. More specifically, it has become clear that the sensitizer of the present invention has, in addition to the anticancer effect as a main effect, a pain-reducing effect, a skin toxicity-reducing effect, or both a pain-reducing effect and a skin-toxicity-reducing effect.

### Brief Description of Drawings

[Figure 1] Figure 1 shows schema of a non-randomised study design.
[Figure 2] Figure 2 shows EQD2 formula used to compare the equivalent RT dose in 2 Gy devided fractions which results in the same log cell death as a given schedule.
[Figure 3] Figure 3 shows sequence of ultrasound images of a breast tumor during H₂O₂ administration. In the figure, Fig. 3A shows an ultrasound image of needle entering (an arrowhead) under ultrasound guidance. Fig.3B shows an ultrasound image of H₂O₂ + sodium hyaluronate gel mixture being injected intratumorally. Fig. 3C shows an ultrasound image of breakdown of H₂O₂ (an arrowhead) with formation of echogenic oxygen microbubbles (an arrowhead) within the tumor.
[Figure 4] Figure 4 shows scheme representing scale for patient self-reported tumor pain. In this figure, tumor pain refers to pain experienced at the target tumor site during radiotherapy with/without H₂O₂ injections.
[Figure 5] Figure 5A shows tumor volume changes in human subjects receiving treatment. In this figure, Fig 5A shows the box plot showing the cumulative fold decrease (log 2 transformed) for tumor volumes of all 12 patients. Fig 5B shows waterfall plot showing % tumor volume change up to 12 months post-RT normalized to baseline tumor measurement. The data represents tumor measurements at 9 and 12 months post-RT for 3 and 8 subjects respectively.
[Figure 6] Figure 6 shows clinical photographs of patient of the suject No. 10 in the Example 6 (6-2). In this figure, Fig 6A shows the clinical photograph of left breast of patient of the suject No. 10 with fungating tumor (baseline). Fig 6B shows the clinical photograph of left breast of patient of the suject No. 10, 12 months post-treatment with H₂O₂ + RT.
[Figure 7] Figure 7 shows scanning images of ¹⁸ F-FDG PET scans of patient of the suject No. 8 in the Example 6 (6-2). In this figure, Fig 7A shows the high tracer uptake in left breast tumor at baseline and Fig 7B shows complete metabolic remission at 12 months post-treatment.

### Description of Embodiments

### Definitions

For convenience, certain terms used in this application are collected here. Unless defined otherwise, all technical and scientific terms used herein have the same meaning as commonly understood by those ordinarily skilled in the art to which this invention belongs. Unless the context clearly defined otherwise, the singular forms "a," "an," and "the" include plural references.

Although the numerical value ranges and parameters set forth herein are approximations, the numerical values set forth in the specific Examples are described as precisely as possible. However, any numerical value inherently contains certain errors necessarily resulting from the standard deviation found in their respective testing measurements. Also, the term "about" means within an acceptable standard error, as considered by those ordinarily skilled in the art.

Embodiments of the present invention will be described below. The following embodiments are examples, and the scope of the present invention is not limited to those described in the following embodiments. Note that descriptions of similar contents are omitted as appropriate in order to avoid repetitive complication.

The sensitizer for anti-cancer therapy of the present invention is characterized by comprising a combination of 0.01-3.5 (v/v) % of component (a) hydrogen peroxide and 0.1-10 (v/v) % of component (b) hyaluronic acid or a salt thereof, wherein the dose of sensitizer administered is selected from the range of 1.2 mL to 12.0 mL in accordance with size of tumor.

The sensitizer for anti-cancer therapy according to the invention is a pharmaceutical composition that can be directly injected into the tumor tissue on a tumor site prior to, or simultaneously with, anti-cancer therapy to enhance the effect that anti-cancer therapy has on the tumor site.

In the present invention, "anti-cancer therapy" may be any anticancer treatment method such as radiotherapy (irradiation), and anti-cancer chemotherapy.

In the present invention, "radiotherapy" refers to a treatment method which irradiates the tumor site to cause the DNA damage on a tumor cell, resulting in cell death of the cancer cells. In this invention, "radiotherapy" can be conducted in combination with "anti-cancer chemotherapy" as described below. In radiotherapy, X-rays, electron beams, proton beams, heavy particle beams, α (alpha) rays, β (beta) rays, γ (gamma) rays and the like can be used as radiation. Radiotherapy may be delivered by an external irradiation method in which radiation is emitted from outside the body, or a sealed brachytherapy method in which a radiation source is directly inserted into a body tissue or a cavity such as the esophagus or uterus for treatment. In the present invention, radiotherapy may be delivered either of external irradiation method or the sealed brachytherapy method, or may be conducted by a combination of the external irradiation method and the sealed brachytherapy method.

In this invention, "anti-cancer chemotherapy" refers to all treatments based on anti-cancer agents (carcinostatic agents). In this invention, "anti-cancer chemotherapy" can be conducted in combination with "radiotherapy" as described above. Effect of radiotherapy by linear accelerator based radiotherapy of cancer cells has around a 70% dependency on the production of reactive oxygen species such as hydroxyl radicals. And hypoxia in tumor tissue affects the production of reactive oxygen species. From such characteristics, dissolution of hypoxia is effective to improve the effect of bot the radiotherapy and chemotherapy. Therefore, a substance that increases the sensitivity of the effect of the radiotherapy at the same time increases the sensitivity of the effect of the anti-cancer chemotherapy.

In the present invention, "tumor" for using the sensitizer in the present invention may be any tumors in general that forms a tumor mass (solid tumor) other than hematological tumors, regardless of whether benign or malignant, or epithelial or non-epithelial. Among such tumors, malignant tumors are mainly assumed as target tumors to which the sensitizer of the present invention is administered. In addition, although the tumor is not particularly limited in sensitivity to anticancer therapy, the sensitizer of the present invention can be used against tumors that have undergone radiation therapy and/or anticancer chemotherapy in the past but are resistant to therapy.

In the present invention, "tumor site" refers to a tumor tissue mainly formed by tumor cells. The tumor tissue also includes tissue in which tumor cells and normal cells and/or normal tissue are present together. When tumor cells and normal cells and/or normal tissue are present together, the ratio of volume or number of tumor cells relative to that of normal cells and/or normal tissue is not particularly limited.

Therapeutic effects of anticancer therapy with or without the sensitizer of the present invention can be evaluated by using general assessment methods for tumor treatment, such as the percentage of tumor tissue shrinkage, patient survival rate, and recurrence rate after treatment.

As mentioned above, the sensitizer of the present invention is a composition comprising a combination of component (a) and component (b). Here, "a combination" is used to mean that the sensitizer of the invention that is used in the present invention:
(i) in a state in which both the component (a) and the component (b) are included from the beginning (combination preparation);
(ii) for sale as a combination (a kit) including a separately packaged formulation containing the component (a) and a separately packaged formulation containing the component (b); or
(iii) in a formulation containing the component (a) and a formulation containing the component (b) that are separately packaged and are in separate market distribution channels and are combined at time of use.

That is, in this invention, " the sensitizer for anti-cancer therapy comprising a combination" means that the sensitizer for anti-cancer therapy that is finally used (referred to in this invention as the "final formulation") may include both the component (a) and the component (b), with the form at the sales and distribution stage being immaterial.

### Component (a)

The sensitizer for anti-cancer therapy of the present invention is characterized by comprising 0.01-3.5 (w/v) % of the component (a) hydrogen peroxide (H₂O₂) as an ingredient.

"Hydrogen peroxide" as used in the present invention refers to hydrogen peroxide molecules (H₂O₂; molecular weight of 34) unless otherwise specified.

In addition, "aqueous hydrogen peroxide solution" refers to a solution obtained by dissolving hydrogen peroxide in distilled water such as the Japanese Pharmacopoeia or the like unless otherwise specified. In this specification, % (w/v) refers to weight/volume percent concentration unless otherwise specified.

### Component (b)

The sensitizer of the invention is characterized by comprising 0.1-10 (w/v) % of the component (b), hyaluronic acid or a salt thereof, as an auxiliary component. The concentration of component (b) hyaluronic acid or a salt thereof contained in the sensitizer of the invention is not limited, and may be preferably in the range of 0.1 to 5 % (w/v), more preferably in the range of 0.1 to 3 % (w/v), still more preferably, the hyaluronic acid or a salt thereof may be used from the range of 0.3 to 1 % (w/v).

The hyaluronic acid used by the invention may be derived from any source; it may be extracted from animal tissues or manufactured by a fermentation method. From the viewpoint of safety and manufacturing stability, it is preferably manufactured by a fermentation method. There is no particular limitation on the strain used in the fermentation method, and any hyaluronic acid producing microorganism desired being usable may include, such as, for example, a hyaluronic acid producing organism isolated from nature that belongs to a genus such as Streptococcus, the Streptococcus equi FM-100 described in JP63-123392A (Fermentation Research Institute Bacillus Deposit No. 9027) or the Streptococcus equi FM-300 described in JP2-234689A (Fermentation Research Institute Bacillus Deposit No. 2319).

Heretofore, hyaluronic acids with various molecular weights have been conventionally known. While it is not a limitation, the present invention can use hyaluronic acid with a mass average molecular weight normally about 500,000 to about 10 million, preferably about 500,000 to about 8 million, and more preferably about 500,000 to about 5 million.

The weight average molecular weight of the hyaluronic acid can be measured by the SEC-MALLS method using a size-exclusion chromatogram (SEC) coupled to a multi-angle laser light-scattering detector (MALLS) (see, for example, C. Yomota, Bull. Natl. Inst. Health Sci., 121, 030-033 (2003)).

The hyaluronic acid that is an object of the invention may be crosslinked hyaluronic acid. Here, the crosslinked hyaluronic acid is a macro-molecule with a three-dimensional mesh structure that forms a gel that swells in the medium. That is, the crosslinked hyaluronic acid has a hydrogel morphology that swells in a physiologically permissible medium.

As an example of the crosslinked hyaluronic acid, there can be mentioned a crosslinked hyaluronic acid formed from a hyaluronic acid with a weight average primary molecular weight that is greater than 800,000. The crosslinked hyaluronic acid is characterized in that, when it is severed at the crosslink point, it forms a straight-chain hyaluronic acid with a weight average molecular weight that is greater than 800,000. The weight average molecular weight and the degree of branching of the hyaluronic acid produced by cutting at the crosslink point can be readily measured by GPC-MALLS (multi-angle light scattering) using a multi-angle laser light-scattering detector (MALLS) and a differential refractive index detector with a gel permission chromatogram (GPC).

The crosslinked hyaluronic acid used by the invention may be one in which the crosslink point is hydrolyzable. Here, a crosslink point that is hydrolyzable means that the crosslink point is apt to degrade before degradation of the main chain of the hyaluronic acid, under physiological conditions such as at 37 degree C and a pH of 7.4, in a physiological saline solution. While crosslink structures with a hydrolyzability that is superior to the main chain degradation of the hyaluronic acid include carbamate bonds, hydrozone bonds, hydrazide bonds, phosphate and ester bonds, and the most typical structures are ester bonds.

Examples of the crosslinked hyaluronic acids with an ester bond crosslinked structure include esters of carboxyl group of hyaluronic acid and polyhydric alcohol, esters of hydroxyl group of hyaluronic acid and polyhydric carboxylic acid, esters of carboxyl group of hyaluronic acid and polyhydric epoxide, and so forth. The crosslinked hyaluronic acid includes a crosslinked hyaluronic acid in which there is a direct ester bond between the carboxyl group and the hydroxyl group of the hyaluronic acid (called "a bridge ester with a self-crosslinked ester bond", or "a hyaluronic acid with a self-crosslinked ester bond").

A hyaluronic acid with self-crosslinked ester bonds can be manufactured by conventionally known methods. For example, a hyaluronic acid with self-crosslinked ester bonds with a part or all of the carboxyl groups esterified with the alcoholic functions of the same polysaccharide chain or other polysaccharide chains can be manufactured as described in EP 0341745 B1; or WO 99/10385 describes a method in which a solution of a hyaluronic acid is acidified, and the solution is frozen and thawed at least once to thereby prepare a hyaluronic acid with self-crosslinked ester bonds; or WO 01/57093 describes a method, in which, without freezing, a hyaluronic acid and an acidic solution are mixed together to produce a 5% or higher concentration and the state of coexistence is maintained to thereby prepare a hyaluronic acid with self-crosslinked ester bonds.

Because the natural hyaluronic acid released by hydrolysis of the hyaluronic acid with self-crosslinked ester bonds is metabolized by physiological metabolic pathways, it is considered to be safer than a crosslinked hyaluronic acid manufactured by other crosslinking reactions.

Examples of a crosslinked hyaluronic acid includes a hyaluronic acid in which the carboxyl group of the hyaluronic acid is crosslinked to the hydroxyl group of the same hyaluronic acid molecules, and/or to the hydroxyl group of different hyaluronic acid molecules (see, for example, JP 2003-252905 A).

The degree of crosslinking of a crosslinked hyaluronic acid, for example, the amount of intermolecular ester bonding of the molecules to be introduced, can be arbitrarily controlled according to the purpose of the crosslinked hyaluronic acid or the required properties thereof. The amount of ester bonding can be defined as the ratio to the total number of carboxyl groups in the hyaluronic acid. In the sensitizer for anti-cancer therapy of this invention, the hyaluronic acid is used preferably as a water solution or a water swelling gel. When the hyaluronic acid is used in the form of an aqueous solution, while there is no limitation, it is preferable to use a crosslinked hyaluronic acid in which the amount of intermolecular ester bonding is less than 0.5%. Also, when the hyaluronic acid is used as a water swelling gel, while there is no limitation, it is preferable to use a crosslinked hyaluronic acid in which the amount of intermolecular ester bonding is about 0.5% to about 1%. The molecular structure of the crosslinked hyaluronic acid can be confirmed using NMR (Carbohydr. Res. Vol 245, p 113-128, 1993; Macromolecules Vol 29, p 2894-2902, 1996).

A crosslinked hyaluronic acid can be prepared by, for example, acidifying an aqueous solution of hyaluronic acid and converting the dissociated carboxyl groups to the acid form. Because, under high-temperature conditions, the deacetylation reaction of an N-acetyl-D-glucosamine unit competes with the crosslinking reaction, it is desirable that the acidic treatment of the hyaluronic acid aqueous solution is performed at a low reaction temperature (see JP H01-266102 (A)). The reaction temperature used to give precedence to crosslink formation is preferably not higher than room temperature, and is more preferably not more than 10 degree C. Also, to promote the intermolecular esterification reaction, it is preferable to raise a hyaluronic acid concentration. For example, the hyaluronic acid concentration in the reaction solution is preferably 5 weight percent or above, and more preferably 10 weight percent or above. Also, a substance that catalyses the dehydration-condensation reaction can also be added to the reaction system to promote the intermolecular esterification reaction. An acidic catalyst is generally used as a catalyst for promoting the dehydration-condensation reaction, for which sulfuric acid, hydrochloric acid or an aromatic sulfonic acid derivatives or the like may be used.

When the crosslinked hyaluronic acid is being formed, materials with the excellent biocompatibility like the hyaluronic acid, for example, chondroitin sulfate, carboxymethyl-cellulose, and so forth may be mixed or compounded to form crosslinked hyaluronic acid. Moreover, when forming crosslinked hyaluronic acid, it is also possible to add pharmacologically or physiologically active substances to form a crosslinked hyaluronic acid containing such substances.

The crosslinked hyaluronic acid thus obtained can then be subjected to an operation to remove acid components to adjust the acidity. This removal of acid components is normally performed in an aqueous solvent by, for example, washing or dialyzing. There is no particular limitation on the aqueous solvent that may be used, provided it does not impair the function of the crosslinked hyaluronic acid. Examples of the usable aqueous solvents include water, physiological saline, a phosphoric acid buffer solution and so forth, but it is preferable to use physiological saline or a phosphoric acid buffer solution and the like. When acid form carboxyl groups remain in the washed crosslinked hyaluronic acid, they can be formed into a salt of sodium or the like (salification). The salification method used includes, for example, but are not limited to, a method of using an aqueous solution of sodium hydroxide to adjust the crosslinked hyaluronic acid solution to a pH of around 7, or a method of immersing the crosslinked hyaluronic acid in physiological saline or a phosphoric acid buffer physiological saline solution.

Depending on the purpose, the crosslinked hyaluronic acid thus prepared may be used in solution form, in the solvent immersion state, or in a moist state that includes the solvent, as the material of the sensitizer for anticancer therapy of the invention (hereinafter also referred to simply as "the sensitizer").

The crosslinked hyaluronic acid swells into a gel in a physiologically permissible medium. Therefore, when the sensitizer of the invention is used as an injection, in order to inject it through a needle into the tumor site to be subjected to irradiation or anti-cancer chemotherapy, it is necessary for the crosslinked hyaluronic acid (gel) to be dispersed as a suspension in a physiologically permissible medium. The suspension may be prepared by crushing the crosslinked hyaluronic acid at either the manufacturing or refining process stage thereof, using a crusher such as a mixer or homogenizer. The diameter of the hydrogel particles of crosslinked hyaluronic acid may be arbitrarily adjusted. However, when the crosslinked hyaluronic acid is homogenized after being dispersed in the physiologically permissible medium, the dispersion particle diameter can be readily adjusted normally from about 0.05 to about 2 micro meter.

The equilibrium swelling ratio of the crosslinked hyaluronic acid gel can be arbitrarily adjusted according to the degree of crosslinking of the crosslinked hyaluronic acid. For example, in the case of an equilibrium swelling ratio of 100 times, the concentration of the hyaluronic acid in the physiologically permissible medium will be 1%. In the case of an equilibrium swelling ratio of 10 times, the concentration of the hyaluronic acid in the physiologically permissible medium will be 10%.

In the sensitizer of this invention, as mentioned in the foregoing, the hyaluronic acid may be either an ordinary hyaluronic acid (non-crosslinked), a crosslinked hyaluronic acid, or an arbitrary combination thereof. A hyaluronic acid in a single form or a hyaluronic acid of a single molecular weight may also be used. Further, various crosslinked hyaluronic acids or hyaluronic acids of various molecular weights may be used in combination.

In the sensitizer of this invention, a hyaluronic acid (as a generic concept that includes a crosslinked hyaluronic acid) may be used in the form of a salt. Suitable examples of hyaluronic acid salts are, but are not limited to, alkali metal salts such as sodium, potassium, lithium, and so forth. A sodium salt of hyaluronic acid is preferable.

### Other components

In addition to the above component (a) and component (b), the sensitizer of the invention may include pharmaceutically acceptable physiological saline, a phosphoric acid buffer solution (for example, sodium chloride, sodium dihydrogen phosphate, and sodium dihydrogen phosphate, and the like). While there is no limitation on the humoral property of the sensitizer of the invention, provided it is compatible with the human body, it is preferable to adjust the pH to a range of 6 to 8.5, and more preferably to a range of 6.8 to 7.8.

In the case where the dosage form of the sensitizer of the invention is an injection form, the sensitizer may include isotonizing agents, pH adjustments, and buffer solutions. Examples of usable isotonizing agents include sodium chloride, glycerine, glucose, polyethylene glycol, propylene glycol, D-mannitol, fructose, xylitol, sodium dihydrogen phosphate and sodium phosphate. Preferably, sodium chloride is used. Examples of the pH adjustment may include hydrochloric acid or sodium hydroxide or the like. The pH is adjusted using the pH adjustment as described above to a range of 6 to 8.5, and preferably to a range of 6.8 to 7.8. Examples of the buffer solutions used for maintaining the pH may include phosphoric acid buffer solutions, Tris buffer solutions and acetic acid buffer solutions. Preferably, a phosphoric acid buffer solution is used.

### Dosage form of the sensitizer of the invention

The sensitizer of the invention is a liquid (including a solution, emulsion or suspension) or a gel, which is used as an injection form. Therefore, the sensitizer of the present invention can be directly injected into the tumor site of a subject for anticancer therapy using a syringe or the like or be injected via an angiographic catheter. Specifically, it is preferable to inject intratumorally the sensitizer of the invention using an injection needle, while controlling the injection volume using a syringe or a catheter pump, while observing the state of permeation of the sensitizer into the tissue under the guidance of ultrasonographic examination. The sensitizer can be delivered widely to the tumor tissue under ultrasonographic guidance by changing the depth and direction of the injection needle.

As described above, the sensitizer for the anti-cancer therapy of the invention is in the form of an injection. The injection can be obtained by preparing an aqueous solution using water for injection (distilled water for injection, sterilized water for injection, etc.), an isotonizing agent, a pH adjustment, and a buffer solution and the like, adding the component (a) and the component (b) to the resulting aqueous solution in the proportions of the above-described range, placing the resulting mixture in a container and then sealing the container, followed by sterilization using a high-pressure steam sterilization, autoclave sterilization or the like. It is also possible to prepare the injection comprising a sensitizer of the invention by suitably mixing a formulation containing the component (a) and a formulation containing the component (b) at time of use (a preparation of type prepared just before use), using water for injection (distilled water for injection, sterile water for injection, etc.), if required.

When the sensitizer of the present invention is used in clinical practice, as an example, as the component (a), 0.4 mL of 3% H₂O₂ encapsulated in a syringe (e.g., 2.0 mL sterile ampoules supplied by Stockport Pharmaceuticals, UK), and, as the component (b), 20 mg sodium hyaluronate pre-loaded in a 2.0 mL pre-loaded syringe (e.g., 2.0 mL of sodium hyaluronate OSTENIL^{®} provided by AAH Pharmaceuticals, UK) can be used to prepare the sensitizer of the present invention. The sensitizer of the present invention can be generated at the time of use by mixing these components. In this case, the two components can be mixed under sterile conditions by connecting a syringe filled with hydrogen peroxide solution and a syringe pre-loaded with a solution of hyaluronic acid or a salt thereof via a 2-way tap, and pumping the syringes alternately. When the component (a) (2.0 mL 3% H₂O₂ sterile ampoule supplied by Stockport Pharmaceuticals, UK) and the component (b) (2.0 mL sodium hyaluronate provided by AAH Pharmaceuticals, UK, OSTENIL^{®}) are used to generate the sensitizer of the present invention, 2.4 mL of sensitizer containing 0.5% H₂O₂ can be prepared by mixing these components. In the actual clinical practice, 2.4 mL of the prepared sensitizer can be evenly divided into two syringes, and 1.2 mL of the sensitizer containing 0.5% H₂O₂ can be used as the minimum dosing unit.

### The dose of sensitizer of the present invention

The frequency of administration of the sensitizer of the present invention can be appropriately determined according to the condition of the tumor to be treated. For example, during the tumor treatment period, the sensitizer is injected into the tumor tissue once to 3 times a week, preferably twice a week. In addition, the sensitizer is injected into the tumor 2 to 8 times in total, preferably 4 to 6 times in total, during the tumor treatment period.

The dose of sensitizer administered to the tumor site may be determined within the range of 1.2 mL to 15.0 mL depending on the status of the tumor to be treated. In other word, the dose of sensitizer administered to the tumor site will determined depending on the size of the tumor to be 0.5 to 5 preparations of the sensitizer containing 0.5% H₂O₂ prepared according to the above-mentioned time-of-use preparation example. For example, when using 2.4 mL of of the sensitizer containing 0.5% H₂O₂ in one preparation, the sensitizer can be administered from 1.2 mL (i.e., 0.5 preparation as a minimum dosing unit) to 12.0 mL (i.e,.5 preparations), and when using 3.0 mL of the sensitizer containing 0.5% H₂O₂ in one preparation, the sensitizer can be administered from 1.5 mL (i.e., 0.5 preparation as a minimum dosing unit) to 15.0 mL (i.e,.5 preparations).

In addition, the dose of the sensitizer injected into the tumor can be adjusted in consideration of the size of the tumor mass, the hardness of the tumor mass, the clarity of the tumor boundary, the type of tumor cell to be treated, and the like. For example, the relationship between the size of the tumor mass and the dose is:
(1) For a tumor with a maximum diameter of less than 3.0 cm, the dose of the sensitizer may be determined to the range of 1.2 mL to less than 3.0 mL (corresponding to 0.5 preparation to 1 preparation),(2) For a tumor with a maximum diameter ranging 3.0 cm to less than 6.0 cm, the dose of the sensitizer may be determined to the range of 2.4 mL to less than 6.0 mL (corresponding to 1 to 2 preparations),
(3) For a tumor with a maximum diameter ranging 6.0 cm to less than 10.0 cm, the dose of the sensitizer may be determined to the range of 3.6 mL to less than 9.0 mL (corresponding to 1.5 to 3 preparations), and
(4) For a tumor with a maximum diameter of 10.0 cm or more, the dose of the sensitizer may be determined to the range of 3.6 mL to 15.0 mL (1.5 to 5 preparations). Tumor size can be measured according to RECIST (Response Evaluation Criteria in Solid Tumors) guidelines.

The sensitizer of the present invention was originally developed to enhance the anti-cancer effect of anti-cancer therapy, and has been known to have an anti-cancer effect. In this study, as results of actual application of the sensitizer of the present invention to patients, it has been clarified that the sensitizer of the present invention exhibits a size-reduction effects of the tumor, which may result in a pain-reducing effect, a dermatological toxicity-reducing effect, or both the pain-reducing effect and the dermatological toxicity-reducing effect, in addition to the anti-cancer effect. It is considered that the sensitizer of the present invention enhances the anti-cancer effect of anti-cancer therapy such as radiation therapy or anti-cancer chemotherapy and reduces the size of the tumor cell mass, resulting in reduction of pain caused by the tumor cell mass. In addition, it is possible to, by using the sensitizer of the present invention in anti-cancer therapy, reduce the intensity of anti-cancer therapy for obtaining the same outcome as when only the anti-cancer therapy is performed. As a result, it is considered that, for example, when radiotherapy is applied as anti-cancer therapy, the radiation dose can be reduced by using the sensitizer of the present invention, resulting in reduction of the skin toxicity generated by irradiation.

### Anti-cancer therapy in combination with the sensitizer of the present invention

The sensitizer of the present invention is not used alone, but is used in combination with anti-cancer therapy used in clinical practice, such as radiation therapy or anti-cancer chemotherapy.

In the case where the sensitizer of the invention is used as the sensitizer for radiotherapy, during radiotherapy, the sensitizer of the invention is applied to a tumor site prior to irradiation. It can be preferably used in radiotherapy of tumors that are radioresistant.

Examples of tumors that are radioresistant include tumors that have many hypoxic tumor cells and tumors that have a lot of anti-oxidative enzyme. The biggest problem in the cancer radiotherapy, which currently is conducted mainly with linear accelerators, is the presence of radioresistant cancer cells. The radioresistant tumor tissues are mostly in hypoxic regions and exhibit resistance to radiotherapy. It is said that, in a hypoxic conditions, the radiation-resistance of these cell is caused because DNA damage induced by the radiation is not fixed by oxygen and reactive oxygen species produced in the cancer cells by the radiation are eliminated by anti-oxidative enzymes, which is said to make it difficult to induce apoptosis in these cells. Specific examples of tumors that are radioresistant include malignant melanomas, malignant glioblastomas and various types of sarcomas such as osteosarcomas, as well as nearly all types of locally advanced neoplasms that have grown to several centimeters or more.

In the case where the sensitizer of the invention is used as the sensitizer for radiotherapy, radiotherapy can be implemented by first applying the sensitizer of the invention to the target tumor area and then irradiate the affected part with X-rays or an electron beam. The dosage and usage of the radiation therapy can be decided as appropriate depending on factors such as the type of radiation, the physical condition of the patient to be treated, and the condition of the tumor to be treated, and can be applied, for example, daily, once every two days, once every three days, once a week, once every 10 days and so on. In the case of daily radiation therapy, the dose per fraction of radiation therapy can be appropriately selected from the range of 1.75 to 3.75 Gy. In the case of daily radiation therapy, the total dose can be appropriately selected from the range of 31.5-67.5 Gy. In the case of daily radiation therapy, period of the radiation therapy can be appropriately selected from the range of 16 to 20 days. In the case of radiation therapy once, twice or three times a week, the dose per dose in radiation therapy can be appropriately selected from the range of 5.0 to 7.0 Gy. In the case of radiation therapy once, twice or three times a week, the total weekly dose can be appropriately selected from the range of 5.0 to 21.0 Gy. In the case of radiation therapy once, twice or three times a week, the total number of radiation treatments can be appropriately selected from the range of 4 to 8 times, preferably 6 times. Radiation therapy is performed within 24 hours, preferably within 12 hours, more preferably within 6 hours, and even more preferably within 2 hours, from direct injection of the sensitizer into the tumor.

In addition, in the case where the sensitizer of the invention is used as the sensitizer for anti-cancer chemotherapy, the sensitizer of the invention may be applied to the tumor site to be treated (preferably by injection), prior to anti-cancer chemotherapy (administration of anti-cancer agent). Preferably, it can be used on tumors that are difficult to treat with anti-cancer chemotherapy or which are relatively large. For example, many solid tumors exhibit resistance to anti-cancer chemotherapy, such as stomach cancer, non-small-cell lung cancer, colorectal and rectal cancer, liver cancer, pancreatic cancer, uterine cancer, cancer of the esophagus and breast cancer. Nearly all locally advanced solid tumors which are advanced locally from these solid tumors are resistant to anti-cancer chemotherapy. For example, the anti-cancer chemotherapy is performed within 24 hours, preferably within 12 hours, more preferably within 6 hours, and even more preferably within 2 hours, from direct injection of the sensitizer into the tumor.

In the case where the sensitizer of the invention is used as the sensitizer for anti-cancer chemotherapy, anti-cancer chemotherapy can be implemented in parallel with the administration of anti-cancer agents (before, after or at the same time as the administration of the anti-cancer agents), by applying the sensitizer of the invention to the target tumor site. The dosage and usage of the anti-cancer chemotherapy can be appropriately determined depending on factors such as the type of anti-cancer drug, the physical condition of the patient to be treated, the condition of the tumor to be treated, and so on. As described above, the sensitizer is administered by intratumoral injection using a needle or a catheter guided by ultrasonographic examination. While the sensitizer injection conditions may differ depending on the degree of advancement of the tumor and its size and the like, a method may be used in which normally each injection is from 1 mL to 5 mL, preferably 3 mL. The dosing schedule may be the same as the radiation therapy schedule.

### Other embodiments of the sensitizer of the present invention

As other embodiments of the present invention, there is also provided a tumor treatment method using a combination of radiation therapy or anticancer chemotherapy and the sensitizer of the present invention. The treatment method of the present invention includes the step of administering the sensitizer of the present invention to the tumor, and the step of performing radiation therapy or anticancer chemotherapy.

### Examples

### Example 1: Study design

In this example, a systematically conducted study was performed that tested intratumoral H2O2 injection in combination with RT in locally advanced breast cancer.

The primary objective of this study was to assess the safety and tolerability of H₂O₂ injections with moderately hypofractionated RT. Secondary endpoints also included the proportion of subjects requiring additional pain medication, the incidence of ≥ grade 3 skin toxicity, and the tumor response assessment.

This non-randomised study was conducted to subjects with locally advanced or locally recurrent breast cancer (with or without metastases) to whom RT was indicated for loco-regional disease control. The subjects were either inoperable due to comorbidities or the extent of local disease, or were not suitable for primary breast surgery due to the presence of metastatic disease.

This trial was conducted at the Royal Marsden NHS Foundation Trust (CCR4502). Approvals by the Research Ethics Committee (REC) and the UK Medicines and Healthcare products Regulatory Agency (MHRA) was obtained prior to trial commencement (IRAS 203161, REC 16/LO/1566, EudraCT 2016-000833-40). Monitoring was undertaken by the Clinical Trials Unit at The Royal Marsden NHS Foundation Trust.

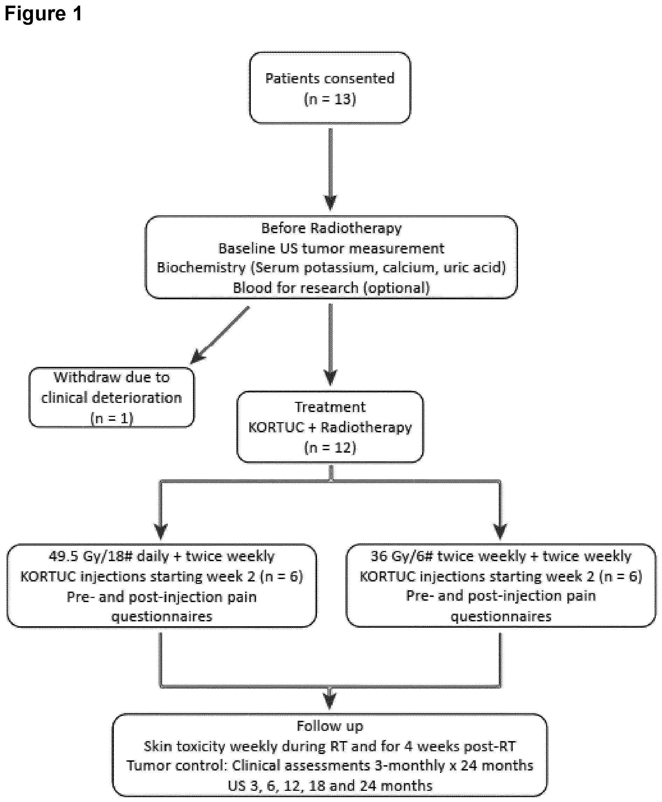

The trial schema is shown in Figure 1. Fig. 1 shows a scheme of a non-randomised study design testing intratumoral administration of H₂O₂ in sodium hyaluronate gel in combination with two radiotherapy fractionation schedules in subjects with locally advanced breast cancer, and corresponding follow up schedule. In this figiure, each symbol depicts the following: #- radiotherapy fraction; US- ultrasound; RT-radiotherapy.

Eligible subjects were 18 years of age or older, had histologically confirmed breast cancer, required breast RT for local control and/or palliation of loco-regional symptoms, and had at least one breast tumor measuring ≥3 cm in diameter in a superficial location accessible for injection. Any combination of oestrogen receptor (ER), progesterone receptor (PR) and HER2 expression was allowed. Exclusion criteria included history of RT to the breast and concomitant biological therapies other than trastuzumab, pertuzumab and denosumab. Female subjects of child-bearing age was excluded. Subjects whose anatomic location of the breast tumor prevented safe access for intratumoral injection, such as the subjects who bear the breast tumor proximity to blood vessels or brachial plexus, were excluded.

According to previously published data, 30-100% of subjects experienced pain described as no worse than `mild' (or CTCAE grade 1) for several hours following injection. One case of tumor lysis syndrome (mild) was reported in the Japanese literature in a total of 139 breast cancer subjects. Given this knowledge of the safety of H₂O₂ plus RT, this trial required 12 subjects to be recruited. Subjects treated with once-daily and twice-weekly fractioned doses of RT were analysed as a single stratum, enrolled in the study, and all subjects who received at least one dose of intratumoral H₂O₂ are defined as the study population. Tumor volumes were calculated using 3-dimensional measurements obtained from US scans.

### Example 2: Drug formulation

This example explains drug formulation used in the study.

A 0.5% H₂O₂ solution used as a sensitizer of the present invention was prepared by mixing 0.4 mL of 3% H₂O₂ (2.0 mL sterile ampoules supplied by Stockport Pharmaceuticals, UK) with 2.0 mL OSTENIL^{®} (20 mg sodium hyaluronate in a 2.0 mL pre-loaded syringe provided by AAH Pharmaceuticals, UK).

The low molecular weight of H₂O₂ (34 g/mol) ensures rapid equilibration of the drug within the gel. The mixture is a colourless, viscous solution (pH 6.8-7.8), stored at room temperature and stable for 2 hours after preparation, as determined by viscosity measurements (performed by Stockport Pharmaceuticals, UK). The gel allows the release of H₂O₂ over at least 24 hours, as evidenced by generation of oxygen microbubbles upon injection, a feature that provides a strong rationale for twice-weekly administration during RT.

In the trial, the drug and gel were mixed under sterile conditions using two syringes connected via a 2-way tap. Once mixed, each syringe contained a total of 2.4 mL of 0.5% H₂O₂, the contents of both syringes are the volume required for tumors measuring 30-60 mm in diameter.

### Example 3: Radiotherapy

This example explains radiotherapy conducted for each subject in the study.

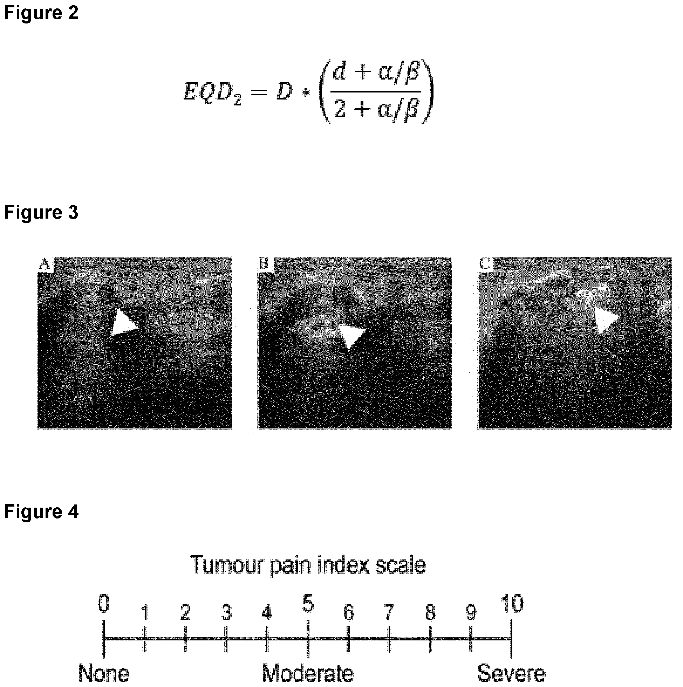

Of the 12 subjects recruited in Example 1, six subjects received 49.5 Gy in total in 18 daily fractions of 2.75 Gy, and 6 subjects were treated with 36 Gy in total in 6 twice-weekly fractions of 6 Gy, to the whole breast or to the whole breast and locoregional lymph nodes. The equivalent RT dose expressed in conventional 2 Gy fractions (EQD2) (Figure 2) (where D= total RT dose, d= dose per fraction, and the α/*β* ratio is a measure of fraction size sensitivity) was 57 Gy and 65 Gy for these two schedules respectively.

Subject on the 6 Gy twice-weekly schedule (the number of subject is 1 out of the 12 above), requiring lymph node irradiation in addition to the breast, were irradiated to a total dose of 30 Gy in 5 twice-weekly fractions of 6 Gy to the brachial plexus regions , in order not to exceed the permissible dose for brachial plexus tolerance dose according to standard institutional guidelines.

The RT schedule was selected according to the subject's performance status and comorbidities, with appropriate subjects being selected for the daily treatment schedule. RT was delivered using a linear accelerator with 6-10 MV photons, and 3D-planning using data from a CT planning scan, and standard tangentially opposed fields. Subjects were simulated and treated in the supine position on a breast board with both arms abducted. The clinical target volume (CTV) comprised the entire ipsilateral breast, including the deep fascia, but excluding underlying muscle or overlying skin (if not involved with disease). The RT dose was defined as the 100% isodose, ensuring the target volume was within the 95%-107% isodose lines.

Organs at risk including the heart, lung and contralateral breast were outlined and standard guidelines for dose tolerances followed. A standard treatment verification protocol consisting of daily imaging for the first 3 days was used, and subsequent weekly imaging was used. In cases where there was skin infiltration by tumor, treatment included 5 mm wax bolus administered throughout RT to maximise dose to skin, in keeping with standard practice. In subjects treated with 49.5 Gy in 18 fractions, a sequential boost dose to the tumor bed (13.35 Gy in 5 daily fractions using mini-tangential opposed beams or a directly applied electron beam) was allowed, but this needed to be declared at time of trial entry.

A tumor bed irradiated with boost dose increased the EQD 2 to that comparable with 36 Gy in 6 fractions and with dose intensities previously reported in earlier cohorts of subjects treated with the same drug preparation.

Table 1 summarizes the subjects' demographics, tumor characteristics, prior treatment and RT target volume.

**[Table 1]**

| **Summary of patient demographics, tumor characteristics, previous lines of treatment and RT treatment volumesPatients** | | | | | | | |
|---|---|---|---|---|---|---|---|
| **Patient** | **Age** | **Sex** | **PS (ECOG*)** | **Baseline TNM stage** | **Tumor Phenotype** | **Prior treatment (no. of lines of therapy)** | **RT target volume** |
| **1** | 77 | F | 1 | T2N1M1 | ER⁺/HER2⁻ | Endocrine (3) | Breast + axillary LN levels I-IV |
| **2** | 69 | F | 0 | T4N0M1 | ER⁺/HER2⁻ | Endocrine (2) | Breast |
| **3** | 79 | F | 3 | T4N0M0 | ER⁺/HER2⁻ | Endocrine (3) | Breast |
| **4** | 80 | M | 2 | T4N0M0 | ER⁺/HER2⁻ | Endocrine (2) | Breast |
| **5** | 89 | F | 3 | T2N0M0 | ER⁺/HER2⁻ | Endocrine (1) | Breast |
| **6** | 78 | F | 2 | T4N1M1 | ER⁺/HER2⁻ | Endocrine (2) Chemotherapy(1) | Breast+ axillary LN levels I-IV |
| **8** | 53 | M | 0 | T2N0M1 | ER-/HER2- | Surgery | Breast |
| | | | | | | Endocrine (3) | |
| | | | | | | Chemotherapy (4) | |
| | | | | | | RT(contralateral) | |
| **9** | 53 | F | 2 | T2N1M1 | ER⁺/HER2⁻ | Surgery | Breast+ axillary LN levels I-IV |
| | | | | | | Endocrine (3) | |
| | | | | | | Chemotherapy (2) | |
| | | | | | | RT(contralateral) | |
| **10** | 45 | F | 0 | T4N1M1 | ER⁺/HER2⁻ | Chemotherapy (1) | Breast+ axillary LN levels I-IV |
| **11** | 75 | F | 3 | T4N1M0 | ER⁺/HER2⁻ | Surgery Endocrine (3) | Breast |
| **12** | 45 | F | 1 | T4N1M1 | ER-/HER2- | Chemotherapy (2) | Breast+ axillary LN levels I-IV |
| **13** | 93 | F | 3 | T2N0M0 | ER⁺/HER2⁻ | None | Breast |

| | | | | | | | |
|---|---|---|---|---|---|---|---|
| Patients 1, 3, 4, 5, 11, and 13 received 36 Gy/6 fractions, and patients 2, 6, 8, 9, 10 and 12 received 49.5 Gy/18 fractions. | | | | | | | |

Thirteen subjects (11 females, 2 males) were recruited to the study between February 2017 and August 2018. All subjects had locally advanced breast cancer or recurrent breast cancer, and were inoperable due to co-morbidities, local extent of disease or metastatic disease. One subject withdrew due to clinical deterioration unrelated to the trial before starting RT and received no H₂O₂, so an additional (13th) patient was recruited. Median age was 77 years (range 45-93). Three subjects were wheelchair-bound due to comorbidities and frailty. Breast tumor stage was T2 in 5/12 subjects and T4 in 7/12 patients. 6/12 had N0 and 6/12 N1 disease (axillary node involvement). Distant metastases was observed in 8/12 subjects. Breast tumor size ranged from 30 mm to 164 mm (maximum dimension). Ten subjects had ER+/HER2-disease and 2 had triple negative disease. There were no subjects with inflammatory breast cancer. All subjects had received 1-4 primer lines of treatment for their breast cancer, and the majority had received prior systemic treatment. Three subjects underwent prior surgery for breast cancer, but had locally recurrent disease. During RT, 7/12 subjects continued taking concurrent endocrine therapy and 2/12 continued bisphosphonate therapy for metastatic bone disease.

Compliance with H₂O₂ injections was 100% in all patients, including 1 with needle phobia. All subjects received RT within the prescribed 1-2 hours after receiving the H₂O₂ injection, with a single exception of a patient given 1 RT fraction before H₂O₂ injection in error. Results are reported at a minimum follow-up of 12 months for all subjects alive at the time of reporting (range 2-24 months). Eleven (11) subjects completed 12 months follow-up, and the subject number 12 died of progressive metastatic disease only 2 months following RT.

### Example 4: Intratumoral injections of H₂O₂ in sodium hyaluronate gel

In this Example, intratumoral injections of H₂O₂ is conducted and the results were monitored.

Transdermal intratumoral KORTUC (the Kochi Oxydol-Radiation Therapy for Unresectable Carcinomas) injections were administered twice-weekly commencing in the second calendar week of RT. Each subject received 4 to 6 doses in total (median = 5 injections), while subjects received smaller number of injection prescribed 6 fractions. The reason for starting KORTUC in the second week was to allow for reduction in tumor interstitial pressure during the first week of RT, enabling technically easier and more tolerable injections for the subject.

Injections were performed (23-gauge needle) under ultrasound (US) guidance by a trained radiologist or radiographer after 0.5% lignocaine injection to anaesthetise the skin. For tumors measuring 30-60 mm in diameter size, two syringes (4.8 mL) of 0.5% H₂O₂ in sodium hyaluronate gel was injected at each time point. Three syringes (7.2 mL) were required for tumors >60 mm in size.

Uniform and accurate delivery under US guidance via 2-3 differently angled needle tracks was aided by the immediate appearance of oxygen microbubbles (see Figure 3C) as H₂O₂ degraded to oxygen and water within the tumor (see Figure 3A). The needle tip was positioned at the deepest side of the tumor and the gel was released slowly whilst withdrawing the needle towards the surface (see Figure 3B). For smaller tumors, it was possible to achieve uniform distribution of the H₂O₂ gel mixture within the tumor via a single skin puncture site and by altering the angle of the needle (working from left to right or top to bottom within the tumor). For some larger tumors (for example > 60 mm) it was necessary to inject the tumor via skin entry points from different directions to ensure uniform distribution of oxygen microbubbles throughout the tumor volume.

The number of needle tracks within the tumor and skin entry points were decided by the radiologist during the ultrasound scan, and were guided by the extent and distribution of the oxygen microbubbles during the injection procedure. If any injected gel be tracked back to the skin surface upon pulling out the needle, it was promptly wiped away with sterile gauze. If subjects had >1 distinct tumors in the breast/axilla, the clinician/radiologist was requested to clearly document the injected lesion (usually the largest) to aid response assessment. RT was delivered within 1-2 hours after H₂O₂ injection.

Within Treatment monitoring in each RT group (daily or twice-weekly fractions), a minimum 1 week interval was defined between the first and second patient, during which acute toxicity data associated with intratumoral injections (pain, skin toxicity, and tumor lysis) was reviewed by an Independent Data Monitoring Committee (IDMC). Based on predetermined criteria, the subject No. 2 and No. 3 in each group and subsequently the subject No. 4, No. 5 and No. 6 in each group were allowed to be treated concomitantly.

### Example 5: Primary endpoint

In this example, it was confirmed that the objective endpoints (the primary endpoints) was achieved in clinical trials in each subject.

Primary endpoints related to the timing, severity and duration of pain post-injection recorded via a self-reported questionnaire completed by subjects at home. An 11-point numerical scale ranging from 0 ("no pain") to 10 ("worst possible pain") were recorded for severity and duration prior to and over 24 hours after each H₂O₂ injection (see Figure 4). Subject-reported scores were used to calculate i) the proportion of subjects with pain scores ≥5 points above baseline after any of the intratumoral injections, and ii) the requirement for additional analgesics.

The pain scores which were collected from the 12 subjects are summarised in Table 2, and based on the information collected from the subjects, grading of tumor pain based on CTCAE v.4.02 criteria shown in Table 3 below were assigned to each subject and respective grading values are filled in the "Maximum RT acute skin toxicity score" column in Table 2.

**[Table 2]**

| **Summary of pain scores and RT acute skin toxicity scores** | | | | | | | |
|---|---|---|---|---|---|---|---|
| **Patient Number** | **Maximum pain intensity** | | **Extra analgesia required** | **Difference in pain score (pre- and post- RT)** | **Effect of pain on ADLs** | **Maximum RT acute skin toxicity score** | **Bolus during RT** |
| | **Score** | **Period** | | | | | |
| 1 | 4 | 2 hrs | N | 3 | Y- housework, Shopping | 3 | Y |
| 2 | 0 | 0 mins | N | 0 | N | 3 | Y |
| 3 | 3 | 30 mins | N | 2.5 | N | 2 | N |
| 4 | 4 | 30 mins | Y | 0.5 | N | 2 | N |
| 5 | 0 | 0 mins | N | 0 | N | 0 | N |
| 6^{a)} | 10 | 6 hrs | Y | 5 | N | 1 | N |
| 8^{b)} | 10 | 6 hrs | Y | 6 | Y-driving | 2 | N |
| 9 | 6 | 5 hrs | N | 4 | N | 2 | N |
| 10 | 8 | 2 hrs | Y | 7 | Y- housework | 3 | Y |
| 11 | 0 | 0 mins | N | 0 | N | 3 | Y |
| 12 | 0 | 0 mins | Y | 0 | N | 3 | Y |
| 13 | 6 | 1 hr | Y | 5 | N | 0 | N |

| | | | | | | | |
|---|---|---|---|---|---|---|---|
| Pain intensity scored from 0-10 via patient self-assessment questionnaires. RT acute toxicity skin sored from 0-5 using CTCAE V4.02 by clinicians. a) patient with needle phobia. b) patient had significant pain in breast prior to H₂O₂ injection and poor compliance with analgesia. ADL: Activities of daily living | | | | | | | |

**[Table 3]**

| **Tumor pain grading (CTCAE)** | **Criteria** |
|---|---|
| 0 | No pain |
| 1 | Mild pain |
| 2 | Moderate pain: limiting instrumental ADL |
| 3 | Severe pain: limiting self-care ADL |
| 4 | - |

As a result, 3 subjects of the 12 subjects experienced grade 1 (mild) tumor pain post injection, and 5 of the 12 subjects experienced grade 2 pain (moderate severity, limiting activities of daily living) as per CTCAE v4.02. The remainder (4 subjects) did not report any additional pain following intratumoral injection. Median pain duration was 60 minutes with an inter-quartile range of 20-120 minutes.

From further detailed examination, 4 subjects of 12 subjects reported pain ≥5 points above baseline during treatment. One subject was taking opiate analgesia (oral morphine) prior to starting radiotherapy to control pain resulting from a fungating breast tumor. Six subjects of 12 subjects required additional analgesia to manage their symptoms (paracetamol and codeine-based). In these cases, management included ensuring compliance with pre-existing analgesics and optimising analgesia and/or anxiolytics for the remainder of their treatment.

### Example 6: Secondary endpoints

In this example, it was confirmed that the items (secondary endpoints) for evaluating effects other than the primary endpoints the clinical trial was achieved in each subject.

Secondary endpoints in this example included RT-induced acute skin toxicity, and tumor response.

### (6-1) Skin toxicity

Skin toxicity was assessed weekly in all subjects during RT period and for 4 weeks post-RT by a member of the clinical team. Standardised proformas recorded the degree of erythema and desquamation of the skin of the breast. In each of the RT groups, if none of the first 3 subjects had a persistent CTCAE (v4.02) skin toxicity ≥ grade 3 six weeks post-RT, the Independent Data Monitoring Committee (IDMC) allowed recruitment of 3 additional subjects to continue within that RT schedule. If moist desquamation was seen beyond skin folds, weekly assessments were continued until severity reduced to ≤ grade 1. The proportion of subjects with ≥ grade 3 skin toxicity at any time from the start of RT to 4 weeks post-RT, and the worst grade of skin toxicity reported from the start of RT to 4 weeks post RT were recorded in these cases. However it was recognised that if cancer has infiltrated skin, subjects would typically experience ≥ grade 3 skin toxicity after RT alone.

Form the information collected from the subjects in this study, radiation dermatitis grade based on CTCAE v.4.02 criteria shown in Table 4 below were assigned to each subject's condition and grading values are filled in "Maximum RT acute skin toxicity score" column in Table 2.

**[Table 4]**

| **Radiation dermatitis grading (CTCAE)** | **Criteria** |
|---|---|
| 0 | No change |
| 1 | Faint erythema or dry desquamation |
| 2 | Moderate to brisk erythema or patchy moist desquamation, mostly confined to skin folds and creases; moderate edema |
| 3 | Confluent moist desquamation, not confined to skin folds; pitting edema |
| 4 | Skin necrosis or ulceration of full-thickness dermis; may include bleeding not included by minor trauma or abrasion |
| 5 | Death |

### Skin toxicity and tumor lysis

The highest grade of skin toxicity reported was grade 3 in 5/12 subjects, grade 2 in 4/12 subjects, grade 1 in 1/12 subjects, and grade 0 in 2/12 subjects (Table 2 and Table 4). All 5 subjects who experienced grade 3 skin toxicity had been treated with bolus during radiotherapy (due to skin infiltration by tumor). There was no suggestion of enhancement of erythema due to local leakage of H₂O₂. The acute radiation skin toxicity observed in the trial was comparable to that expected with standard RT alone, including in subjects with cancer infiltrating overlying skin. There were no cases of tumor lysis syndrome.

### (6-2) Tumor response

Tumor response was assessed at 3, 6, and 12 months post-treatment. At each timepoint, 3-dimensional US measurements were obtained and the tumor volume calculated on the assumption that breast tumors have a hemi-ellipsoid shape, as previously demonstrated. Maximum tumor dimension alone was not considered to be accurately represent tumor response, especially when tumors has 'flattened' after radiotherapy. Tumor volumes were compared to pre-treatment measurements applying RECIST-like principles, with complete response (CR) defined as disappearance of the target lesion, partial response (PR) as at least a 30% reduction in tumor volume, and stable disease (SD) as less than a 30% reduction or 20% increase in tumor volume.

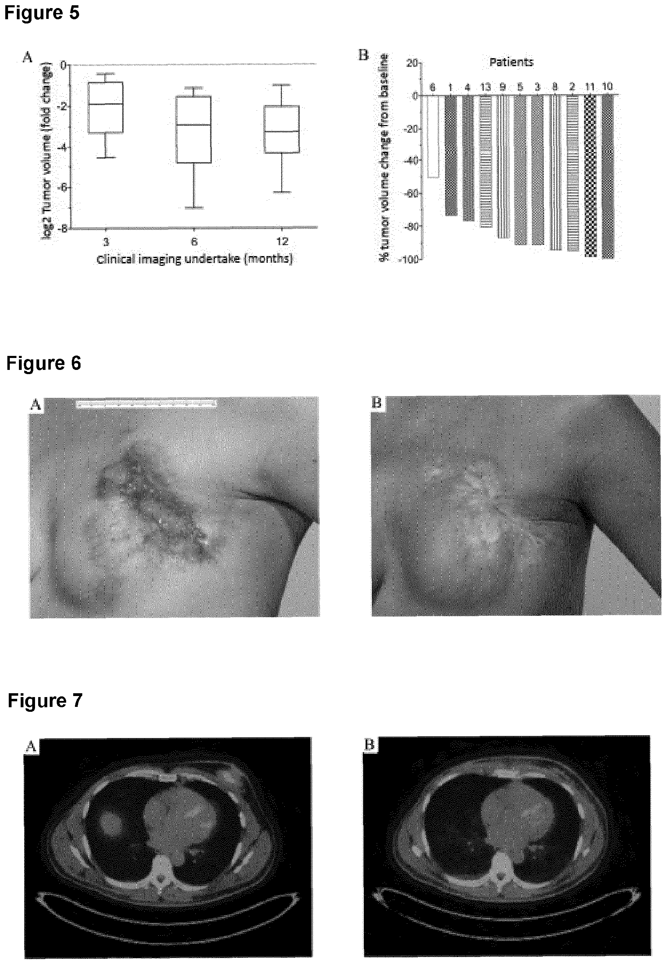

Figures 5A and Table 5 show details of the tumor response based on US measurements at successive time-points post-treatment.

**[Table 5]**

| Tumor volume (at last assessment) and clinical response assessment 3-12 months post-treatment | | | | | | |
|---|---|---|---|---|---|---|
| **Phase I tumor size measurement by US (mm)** | | | | | | **Clinical assessment** |
| **Patients** | **Baseline** | **3 months** | **6 months** | **9 months** | **12 months** | |
| 1 | 42x28x37 | 40x27x30 | 36x22x25 | - | 29x16x25* | PR |
| 2 | 55x30x47 | 28x9x31 | 12x8x28 | 12x11x29 | - | PR |
| 3 | 47x36x30 | 32x17x25 | 26x25x17 | - | 18x14x17 | PR |
| 4 | 29x30x30 | 17x20x17 | 22x20x12 | 18x24x18 | - | PR |
| 5 | 43x17x14 | 31x9x16 | 19x8x8 | 18x7x7 | - | CR |
| 6 | 33x19x41 | 45x15x28 | 41x10x24 | - | 37x15x23 | PR |
| 7 | - | - | - | - | - | - |
| 8 | 49x19x48 | 41x10x33t | 21x14x20 | - | 18x12x11 | CR |
| 9 | 26x24x31 | 14x8x10 | 6x5x5 | - | 16×12×13‡ | PR |
| 10 | 41x14x18 | 8x6x9 | 7x3x5 | - | 0x0x0 | CR |
| 11 | 64x45x65 | 46x15x34 | 39x13x33 | - | 25x4x24 | CR |
| 12 | 164x102x164§ | - | - | - | - | - |
| 13 | 20x18x40 | 23x14x25 | 22x12x19 | - | 18x17x9 | PR |

| | | | | | | |
|---|---|---|---|---|---|---|
| PR: Partial Response CR: Complete Response * KORTUC injected tumor showed partial response, compared to 2 further breast tumors at non-injected sites which demonstrated progressive disease † Patient had complete metabolic response in breast tumor on PET scan (3 months post treatment) which is maintained despite progressive disease outside the breast. ‡ Tumor dimensions increased at 12 months (vs. 6 months) post treatment; US scan shows this is due to fibrosis. § Sarcomatioid breast tumor, measured on CT due to large size. Rapid growth between baseline scan and start of RT, but growth and ulceration stabilised following RT. Patient died 6 weeks post-treatment. | | | | | | |

At the last imaging assessment, percentage tumor volume reduction was between 50 and 100% as shown in Figure 5B. All subjects evaluated in this study maintained loco-regional control in the irradiated target lesion at final clinical follow-up (median 12 months, range 2-24 months). Subject No. 12 died of metastatic disease 6 weeks post-RT and was not evaluable for the 3-month endpoint of tumor response.

As an illustrative example, Figure 6 shows tumor extent in the subject No. 10 before RT and 12 months post-treatment (subject maintained CR at 18 months). Only 1 subject of 12 subjects had >1 distinct tumor lesions within the RT dose. In this subject, only the tumor injected with H₂O₂ showed maintained PR after 12 months, whereas the 2 lesions receiving the same RT alone showed stable disease status (the non-injected lesions acting as internal controls). With regards to tumor response assessment, there were discrepancies in 2 subjects between US measurements and clinical response assessments (Table 5). In the subject No. 9, US measurements between 6 and 12 months suggested an increase in tumor size despite an excellent partial response on clinical examination. Radiologic review of the US images 12 months post-treatment indicated changes consistent with fibrosis rather than active tumor. Similarly, the subject No. 8 demonstrated a complete response on clinical assessment after 12 months, despite the presence of stable measurable disease on US imaging. A staging PET/CT scan performed concurrently confirmed a complete metabolic response in the H₂O₂ + RT-treated breast tumor, as shown in Figure 7.

In summarizing the Examples as described above, it was demonstrated that the study conducted in the Examples raised no concerns relating to local or systemic toxicity when intratumoral H₂O₂ is delivered with RT doses per fraction up to 6 Gy in subjects with locally advanced primary or recurrent breast cancer unsuitable for primary surgery or palliative debulking. The intervention is well tolerated even by the frail, older subjects and those with needle phobia.

Furthermore, in this case where the sensitizer of the present invention was used in combination with the radiotherapy, injection of H₂O₂ started in the second calendar week of radiotherapy ensured that there was no technical problem (i.e. resistance due to tissue compression) to injecting the prescribed volume of drug in any of the patients.

Acute skin toxicity was no different from that expected after the same RT alone. As predicted, grade 3 radiation dermatitis occurred only in those subjects with skin infiltration of tumor (when 5 mm layer of wax 'blanket' ensured 100% prescribed RT dose to skin instead of approximately 70% of prescribed dose in subjects without skin infiltration). Grade 3 skin desquamation was managed with standard supportive measures including barrier creams and dressings, which ensured complete resolution of symptoms in every case.

### Industrial Applicability

The dose and usage of the sensitizer for the anticancer therapy of the present invention can improve the effect of the anticancer therapy (such as radiotherapy or anticancer chemotherapy) and can effectively treat the tumor. More specifically, it has become clear that the sensitizer of the present invention has, in addition to the anticancer effect as a main effect, a pain-reducing effect, a skin toxicity-reducing effect, or both a pain-reducing effect and a skin-toxicity-reducing effect.

## Claims

1. A sensitizer for anti-cancer therapy comprising (a) 0.01-3.5 (w/v) % of hydrogen peroxide and (b) 0.1-10 (w/v) % of hyaluronic acid or a salt thereof, wherein the dose of sensitizer administered is selected from the range of 1.2 mL to 12.0 mL in accordance with size of tumor.

2. The sensitizer according to Claim 1, wherein the sensitizer comprises 0.5 (w/v) % of hydrogen peroxide.

3. The sensitizer according to Claim 1 or 2, wherein the dose of the sensitizer is determined to:
(1) the range of 1.2 mL to less than 3.0 mL for a tumor with a maximum diameter of less than 3.0 cm,
(2) the range of 2.4 mL to less than 6.0 mL for a tumor with a maximum diameter ranging 3.0 cm to less than 6.0 cm,
(3) the range of 3.6 mL to less than 9.0 mL for a tumor with a maximum diameter ranging 6.0 cm to less than 10.0 cm, and
(4) the range of 3.6 mL to 15.0 mL for a tumor with a maximum diameter of 10.0 cm or more.

4. The sensitizer according to any one of Claims 1 to 3, wherein the sensitizer exhibits a pain-reducing effect, a dermatological toxicity-reducing effect, or both the pain-reducing effect and the dermatological toxicity-reducing effect, in addition to the anti-cancer effect.

5. The sensitizer according to any one of Claims 1 to 4, wherein the anti-cancer therapy is radiation therapy or anti-cancer chemotherapy.

6. The sensitizer according to any one of Claims 1 to 5, wherein the sensitizer is directly injected into the tumor tissue once to 3 times a week during the tumor treatment period.

7. The sensitizer according to any one of Claims 1 to 6, wherein the sensitizer is directly injected into the tumor 2 to 8 times in total during the tumor treatment period.

8. The sensitizer according to any one of Claims 1 to 7, wherein the anti-cancer therapy is performed within 24 hours after the direct injection of the sensitizer into the tumor.

9. The sensitizer according to Claim 8, wherein the anti-cancer therapy is applied daily.

10. The sensitizer according to Claim 8 or 9, wherein the dose per fraction in radiation therapy is within the range of 1.75 to 3.75 Gy.

11. The sensitizer according to Claim 8, wherein the radiation therapy is applied once, twice or three times a week.

12. The sensitizer according to Claim 11, wherein the dose per fraction in radiation therapy is within the range of 5.0 to 7.0 Gy.
